# EUROPEAN PATENT APPLICATION

(11) **EP 4 570 222 A1**
(43) Date of publication of application: **18.06.2025**
(21) Application number: 23865323.2
(22) Date of filing: 31.08.2023
(51) Int. Cl.: A61F 13/532, A61F 13/534, A61F 13/539

(54) **ABSORBENT SHEET**

(30) Priority: 16.09.2022 JP 2022148212
(71) Applicant: Zuiko Corporation, Ibaraki-shi Osaka 5670082 (JP)
(72) Inventor: UMEBAYASHI, Toyoshi, Ibaraki-shi, Osaka 567-0082 (JP)
(74) Representative: Hasegawa, Kan
(86) International application number: PCT/JP2023/031979
(87) International publication number: WO 2024/057968

(57) **Abstract**

Provided is an absorbent sheet capable of preventing channel crushing.

The absorbent sheet (10) comprises: a base sheet (12); a plurality of absorbent layers (21 to 23) which contain a superabsorbent polymer resin and which are disposed apart from each other on a main surface (12s) of the base sheet (12); and a covering sheet (14) covering the main surface (12s) of the base sheet (12) and the absorbent layers (21 to 23). This absorbent sheet (10) is configured so that, when the superabsorbent polymer resin in the absorbent layers (21 to 23) swells, the base sheet (12) is more easily deformed than the covering sheet (14).

## Description

### TECHNICAL FIELD

The present invention relates to an absorbent sheet, and more specifically, relates to an absorbent sheet used for absorbing liquid by paper diapers and the like.

### BACKGROUND ART

Absorbent sheets for absorbing liquid are used for paper diapers, incontinence pads, sanitary napkins and the like.

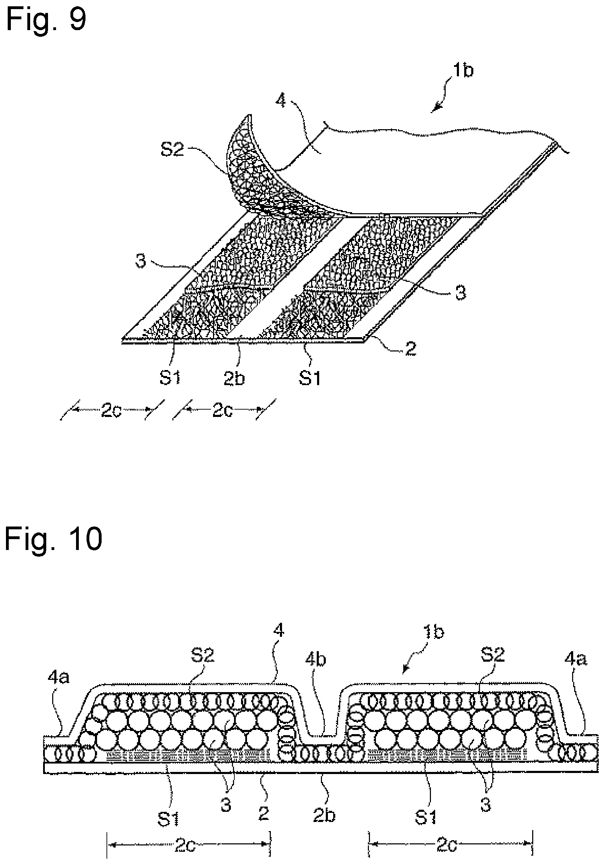

For example, an absorbent sheet 1 shown in Figs. 9 and 10 has a superabsorbent polymer resin disposed on a base sheet. Fig. 9 is a partial cutaway perspective view of the absorbent sheet 1, and Fig. 10 is a cross-sectional schematic view thereof. As shown in Figs. 9 and 10, first hot melt adhesive layers S1 are provided in such a manner as to be separated into right and left parts on a first nonwoven fabric 2, absorbent resin powder layers 3 formed of superabsorbent polymer resin powder are provided in such a manner as to be separated into right and left parts on the right and left first hot melt adhesive layers S1, and further, a second hot melt adhesive layer S2 is provided substantially on the entire surface of the first nonwoven fabric 2. Further, a second nonwoven fabric 4 having substantially the same area and the same configuration as the first nonwoven fabric 2 is provided on the second hot melt adhesive layer S2 (for example, see Patent Literature 1).

### CITATION LIST

### PATENT LITERATURE

[Patent Literature 1] JP 4795612 B2

### SUMMARY OF THE INVENTION

### PROBLEM TO BE SOLVED BY THE INVENTION

The second nonwoven fabric 4 is recessed in an intermediate region 2b between absorbent resin powder existing areas 2c where the absorbent resin powder layers 3 are provided. This forms a channel. When fluid flows along the channel, the area widens where liquid is absorbed by the superabsorbent polymer resin.

However, if the superabsorbent polymer resin swells to crush the channel and this narrows or cuts off the flow path, the liquid absorbing performance deteriorates.

In view of such circumstances, a problem to be solved by the present invention is to provide an absorbent sheet capable of preventing channel crushing.

### MEANS FOR SOLVING THE PROBLEM

To solve the above-mentioned problem, the present invention provides an absorbent sheet configured as follows:

An absorbent sheet is provided with: a base sheet; a plurality of absorbent layers disposed with spacing in between on a main surface of the base sheet and containing a superabsorbent polymer resin; and a covering sheet covering the main surface of the base sheet and the absorbent layers, and is configured so that, when the superabsorbent polymer resin in the absorbent layers swells, the base sheet is more easily deformed than the covering sheet.

According to the above-described configuration, channels are formed between the adjoining absorbent layers. When the superabsorbent polymer resin in the absorbent layers swells, the spacings between the adjoining absorbent layers do not easily change because the covering sheet covering the absorbent layers is less deformable than the base sheet. Consequently, channel crushing can be prevented.

Preferably, the absorbent sheet is configured so that, when the superabsorbent polymer resin in the absorbent layers swells, the base sheet is compressed in a thickness direction and deformed.

In this case, when the superabsorbent polymer resin in the absorbent layers swells, the spacings between the adjoining absorbent layers do not easily change. Consequently, channel crushing can be prevented.

Preferably, the base sheet has a lower rebound than the covering sheet.

In this case, channel crushing can be prevented by configuring the absorbent sheet so that, when the superabsorbent polymer resin in the absorbent layers swells, the base sheet is compressed in the thickness direction and deformed.

Preferably, the covering sheet is bonded to an area of the main surface of the base sheet where the superabsorbent polymer resin is substantially absent.

In this case, when the superabsorbent polymer resin in the absorbent layers swells, the spacings between the adjoining absorbent layers are less likely to change compared with when the covering sheet and the base sheet are not bonded. Consequently, channel crushing can be more reliably prevented.

Preferably, the lateral stress on the base sheet at 5% swelling is lower than the lateral stress on the covering sheet at 5% swelling. Here, "lateral stress at swelling" refers to "shrinkage stress acting in a lateral direction orthogonal to the swelling direction at swelling and the thickness direction" or "tension in the swelling direction per unit length in the lateral direction orthogonal to the swelling direction at swelling and the thickness direction."

In this case, the absorbent sheet can be configured so that, when the superabsorbent polymer resin in the absorbent layers swells, the base sheet is more easily deformed than the covering sheet.

Preferably, the base sheet has a bulky structure where pores are formed between fibers.

In this case, since the base sheet is easily deformed, the absorbent sheet can be configured so that, when the superabsorbent polymer resin in the absorbent layers swells, the base sheet is more easily deformed than the covering sheet.

Preferably, the bulk density of the bulky structure of the base sheet is lower than the bulk density of the covering sheet.

In this case, the absorbent sheet can be configured so that the base sheet is more easily deformed than the covering sheet.

Preferably, the thickness of the covering sheet is equal to the thickness of the base sheet or is smaller than the thickness of the base sheet.

In this case, the entire absorbent sheet can be made thin compared with when the thickness of the covering sheet is larger than that of the base sheet.

### EFFECTS OF THE INVENTION

The absorbent sheet of the present invention is capable of preventing channel crushing.

### BRIEF DESCRIPTION OF THE DRAWINGS

[Fig. 1] Fig. 1(a) is a plan view of an absorbent sheet, Fig. 1(b) is a schematic cross-sectional view taken along the line B-B of Fig. 1(a), and Fig. 1(c) is a schematic cross-sectional view at swelling (first embodiment).
[Fig. 2] Fig. 2(a) is a plan view of an absorbent sheet, Fig. 2(b) is a schematic cross-sectional view taken along the line B-B of Fig. 2(a), and Fig. 2(c) is a schematic cross-sectional view at swelling (first modification).
[Fig. 3] Fig. 3(a) is a plan view of an absorbent sheet, Fig. 3(b) is a schematic cross-sectional view taken along the line B-B of Fig. 3(a), and Fig. 3(c) is a schematic cross-sectional view at swelling (second modification).
[Fig. 4] Fig. 4(a) is a plan view of an absorbent sheet, Fig. 4(b) is a schematic cross-sectional view taken along the line B-B of Fig. 4(a), and Fig. 4(c) is a schematic cross-sectional view at swelling (third modification).
[Fig. 5] Fig. 5(a) is a plan view of an absorbent sheet, Fig. 5(b) is a schematic cross-sectional view taken along the line B-B of Fig. 5(a), and Fig. 5(c) is a schematic cross-sectional view at swelling (fourth modification).
[Fig. 6] Fig. 6(a) is a plan view of an absorbent sheet, Fig. 6(b) is a schematic cross-sectional view taken along the line B-B of Fig. 6(a), and Fig. 6(c) is a schematic cross-sectional view at swelling (fifth modification).
[Fig. 7] Fig. 7(a) is a perspective view of an absorbent sheet on the opposite side of a base sheet relative to one main surface of the base sheet, Fig. 7(b) is a perspective view of the base sheet of the absorbent sheet, and Fig. 7(c) is a perspective view of the absorbent sheet on the opposite side of the base sheet relative to the other main surface of the base sheet (sixth modification).
[Fig. 8] Fig. 8(a) is a perspective view of an absorbent sheet on the opposite side of a base sheet relative to one main surface of the base sheet, Fig. 8(b) is a perspective view of the base sheet of the absorbent sheet, and Fig. 8(c) is a perspective view of the absorbent sheet on the opposite side of the base sheet relative to the other main surface of the base sheet (seventh modification).
[Fig. 9] Fig. 9 is a partial cutaway perspective view of the absorbent sheet (first conventional example).
[Fig. 10] Fig. 10 is a cross-sectional schematic view taken along the line X-X of Fig. 9 (first conventional example).

### MODE FOR CARRYING OUT THE INVENTION

Hereinafter, an embodiment of the present invention will be described with reference to the drawings.

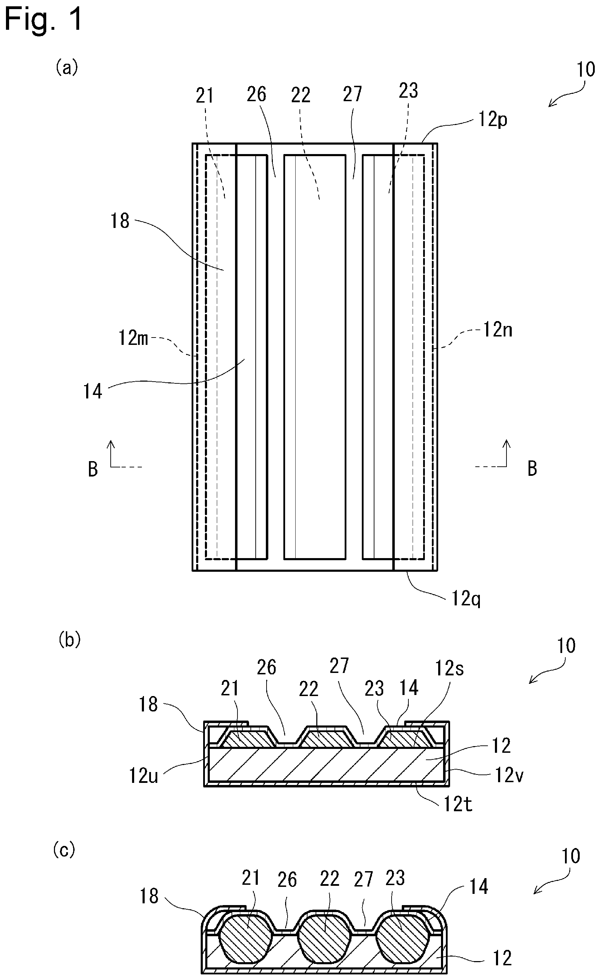

[First embodiment] An absorbent sheet 10 of a first embodiment will be described with reference to Fig. 1. Fig. 1(a) is a plan view of the absorbent sheet 10, Fig. 1(b) is a schematic cross-sectional view taken along the line B-B of Fig. 1(a), and Fig. 1(c) is a schematic cross-sectional view at swelling.

As shown in Fig. 1, the absorbent sheet 10 is provided with: a base sheet 12; a plurality of absorbent layers 21 to 23 disposed apart from each other on one main surface 12s of the base sheet 12 and containing a superabsorbent polymer resin; a covering sheet 14 covering the main surface 12s of the base sheet 12 and the absorbent layers 21 to 23; and a core wrap sheet 18 wrapping the base sheet 12 and the covering sheet 14.

The base sheet 12 is rectangular, and extends in the length direction (the vertical direction in Fig. 1(a)).

The absorbent layers 21 to 23 are formed of a powdery or granular superabsorbent polymer resin disposed so as to have a predetermined thickness in predetermined areas of the one main surface 12s of the base sheet 12. The absorbent layers 21 to 23 are disposed with spacing in between in a width direction intersecting with the length direction of the base sheet 12, and extends in the length direction in parallel to each other. The absorbent layers 21 to 23 are disposed in such a manner that a gap is left between them and edges 12p and 12q and edges 12m and 12n of the base sheet 12.

The covering sheet 14 is formed of a liquid-permeable sheet material, and parts thereof covering the absorbent layers 21 to 23 are raised higher than the part covering the surrounding area of the absorbent layers 21 to 23. This forms groove-like channels 26 and 27 extending in the length direction between the adjoining absorbent layers 21 and 22, and 22 and 23.

The core wrap sheet 18 covers the other main surface 12t and side surfaces 12u and 12v of the base sheet 12, and overlaps with the covering sheet 14 so that the channels 26 and 27 are exposed, thereby wrapping the base sheet 12 and the covering sheet 14 in a C shape. The core wrap sheet 18 may be bonded to the base sheet 12 and/or the covering sheet 14 by an adhesive such as hot melt, supersonic bonding, thermal fusion bonding or the like.

The absorbent sheet 10 is configured so that, when the superabsorbent polymer resin in the absorbent layers 21 to 23 swells, the base sheet 12 is more easily deformed than the covering sheet 14.

For example, as conceptually shown in Fig. 1(c), the absorbent sheet 10 is configured so that, when the superabsorbent polymer resin in the absorbent layers 21 to 23 of the absorbent sheet 10 absorbs liquid and swells, the base sheet 12 is compressed in the thickness direction and deformed.

Specifically, the base sheet 12 is a nonwoven fabric sheet having a bulky structure where pores are formed between fibers along the one main surface 12s, the covering sheet 14 is a nonwoven fiber sheet having no bulky structure, and the bulk density of the bulky structure of the base sheet 12 is lower than that of the covering sheet 14. In this case, when the superabsorbent polymer resin in the absorbent layers 21 to 23 swells, the base sheet 12 is easily deformed in the length direction compared with the covering sheet 14 and the spacings between the adjoining absorbent layers 21 and 22, and 22 and 23 do not easily change, so that channel crushing can be prevented.

The base sheet 12 may be configured so as to have a lower rebound than the covering sheet 14. In this case, when the superabsorbent polymer resin in the absorbent layers 21 to 23 swells, the base sheet 12 is easily deformed in the thickness direction compared with the covering sheet 14 and the spacings between the adjoining absorbent layers 21 and 22 and between 22 and 23 do not easily change, so that channel crushing can be prevented.

The lateral stress on the base sheet 12 at 5% swelling is made lower than that on the covering sheet 14 at 5% swelling.

The thickness of the covering sheet 14 is made equal to that of the base sheet 12 or smaller than that of the base sheet 12.

The basis weight of the covering sheet 14 is made equal to that of the base sheet 12, or is made smaller than that of the base sheet 12.

As an example, the lateral stress at 5% swelling is set so as to decrease in the order of the core wrap sheet 18, the covering sheet 14 and the base sheet 12. In this case, when the superabsorbent polymer resin in the absorbent layers 21 to 23 swells to press the base sheet 12, the covering sheet 14 and the core wrap sheet 18, the base sheet 12 is the most deformable and the core wrap sheet 18 is the least deformable. For this reason, the deformation of the base sheet 12 in the plane direction is restrained by the core wrap sheet 18 and this makes the base sheet 12 easily deformed in the thickness direction, and the spacings between the adjoining absorbent layers 21 and 22, and 22 and 23 do not easily change, so that crushing of the channels 26 and 27 can be prevented.

In another example, the lateral stress on the core wrap sheet 12 at 5% swelling is made lower than that on the core wrap sheet 18 at 5% swelling. In this case, when the superabsorbent polymer resin in the absorbent layers 21 to 23 swells to press the base sheet 12, the covering sheet 14 and the core wrap sheet 18, the core wrap sheet 14 is more deformable than the base sheet 12. For this reason, the base sheet 12 becomes vulnerable to deformation in the plane direction and the spacings between the adjoining absorbent layers 21 to 23 easily increase. Consequently, crushing of the channels 26 and 27 can be prevented.

The thickness of the core wrap sheet 18 is made smaller than that of the base sheet 12.

The basis weight of the core wrap sheet 18 is made smaller than that of the base sheet 12.

The basis weight of the core wrap sheet 18 is made within the range of not less than 20g/m² and not more than 70g/m².

For example, an air-through nonwoven fabric is used for the base sheet 12, a spun lace nonwoven fabric is used for the covering sheet 14, and an SMS nonwoven fabric is used for the core wrap sheet. Here, the SMS nonwoven fabric refers to a three-layered nonwoven fabric where a melt-blown nonwoven fabric is sandwiched between spunbond nonwoven fabrics.

Although a configuration may be adopted such that the covering sheet 14 and the base sheet 12 are not bonded, preferably, the covering sheet 14 and the base sheet 12 are bonded. Specifically, the covering sheet 14 is bonded to, of the one main surface 12s of the base sheet 12, the surrounding area of the absorbent layers 21 to 23 (area where the superabsorbent polymer resin is substantially absent) by an adhesive such as hot melt, supersonic bonding, thermal fusion bonding or the like. When the covering sheet 14 and the base sheet 12 are bonded, the spacings between the adjoining absorbent layers 21 and 22, and 22 and 23 do not easily change compared with when the covering sheet 14 and the base sheet 12 are not bonded, so that crushing of the channels 26 and 27 can be more reliably prevented. The covering sheet 14 may be bonded to the base sheet 12 by applying hot melt to the entire area of the one main surface 12s of the base sheet 12 from above the absorbent layers 21 to 23.

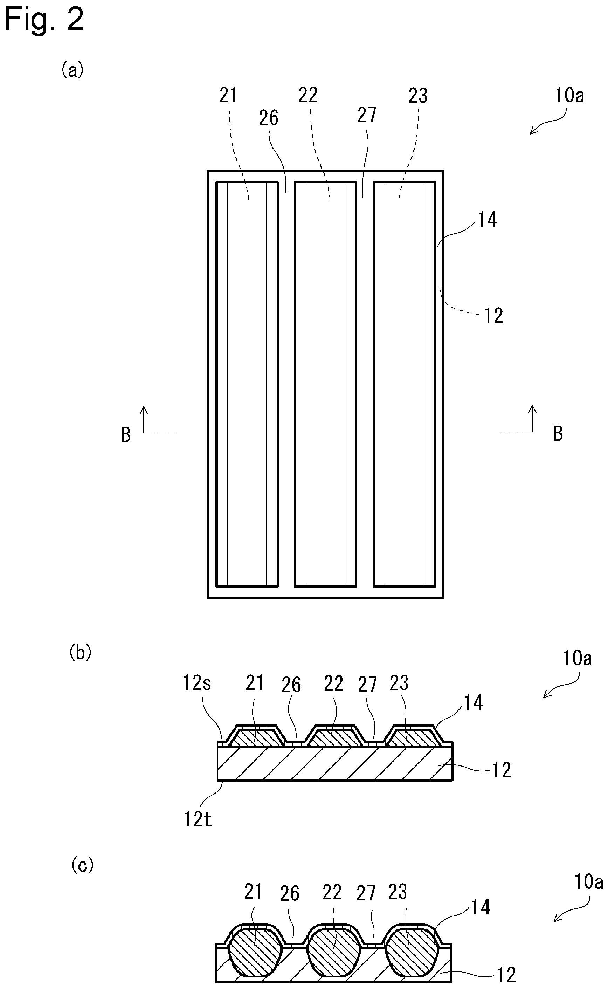

Next, first to seventh modifications will be described with reference to Figs. 2 to 8. In the following, component parts similar to those of the first embodiment will be denoted by the same reference numerals as those of the first embodiment and differences from the first embodiment will be mainly described.

[First modification] A configuration may be adopted such that the core wrap sheet 18 wrapping the base sheet 12 and the covering sheet 14 is eliminated from the absorbent sheet 10 of the first embodiment like an absorbent sheet 10a of a first modification shown in Fig. 2.

For example, as shown in Fig. 2(c), the absorbent sheet 10a is configured so that, when the superabsorbent polymer resin in the absorbent layers 21 to 23 swells, the base sheet 12 is compressed in the thickness direction and deformed. This prevents crushing of the channels 26 and 27.

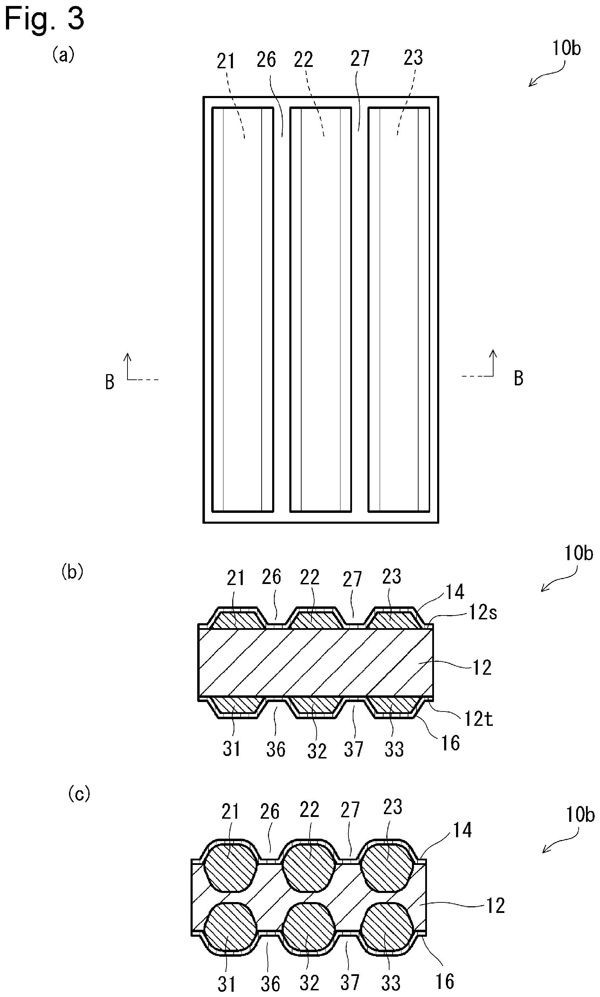

[Second modification] A configuration may be adopted such that channels 26 and 27, and 36 and 37 are formed on both surfaces of the base sheet 12 like an absorbent sheet 10b of a second modification shown in Fig. 3.

As in the first embodiment, the absorbent sheet 10b is configured so that the absorbent layers 21 to 23 are disposed on the one main surface 12s of the base sheet 12, the one main surface 12s of the base sheet 12 and the absorbent layers 21 to 23 are covered by the covering sheet 14 and the groove-like channels 26 and 27 are formed between the adjoining absorbent layers 21 and 22, and 22 and 23.

Unlike the first embodiment, the absorbent sheet 10b is further provided with: a plurality of absorbent layers 31 to 33 disposed apart from each other on the other main surface 12t of the base sheet 12 and containing the superabsorbent polymer resin; and a covering sheet 16 covering the other main surface 12t of the base sheet 12 and the absorbent layers 31 to 33. The absorbent layers 31 to 33 and the covering sheet 16 on the other main surface 12t side of the base sheet 12 are formed of similar materials and configured similarly to the absorbent layers 21 to 23 and the covering sheet 14 on the one main surface side 12s of the base sheet 12, and groove-like channels 36 and 37 are formed between the adjoining absorbent layers 31 and 32, and 32 and 33.

For example, as shown in Fig. 3(c), the absorbent sheet 10b is configured so that, when the superabsorbent polymer resin in the absorbent layers 21 to 23 and 31 to 33 swells, the base sheet 12 is compressed in the thickness direction and deformed, so that crushing of the channels 26 and 27, and 36 and 37 can be prevented.

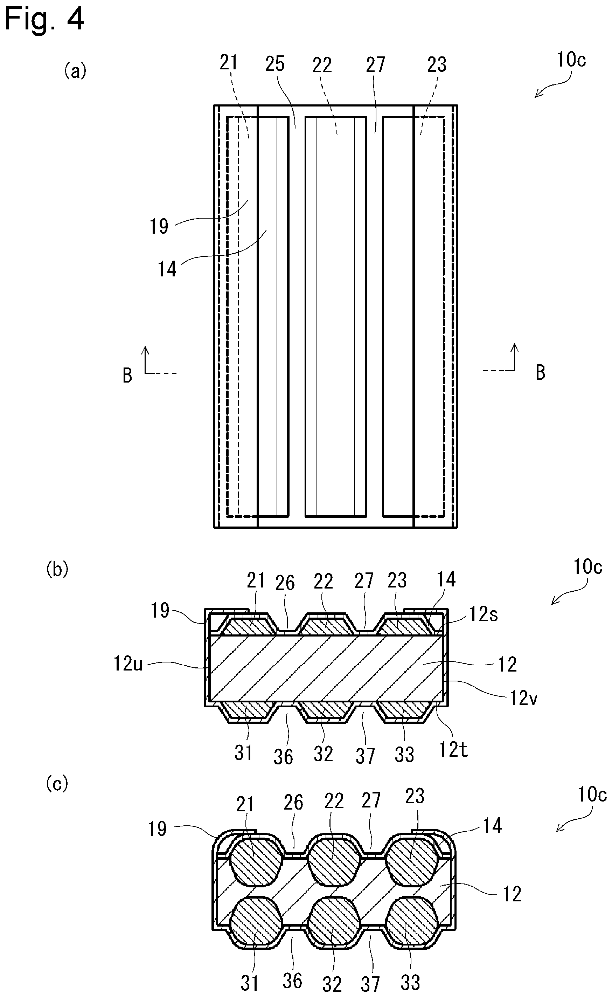

[Third modification] Like an absorbent sheet 10c of a third modification shown in Fig. 4, the covering sheet 16 of the second modification may be changed to a core wrap sheet 19.

The core wrap sheet 19 covers the other main surface 12t of the base sheet 12 and the absorbent layers 31 to 33 like the covering sheet 16 of the second modification, covers the side surfaces 12u and 12v of the base sheet 12 like the core wrap sheet 18 of the first modification, and overlaps with the covering sheet 14 so that the channels 26 and 27 are exposed, thereby wrapping the base sheet 12 and the covering sheet 14 in a C shape. The core wrap sheet 19 may be bonded to the base sheet 12 and/or the covering sheet 14 by an adhesive such as hot melt, supersonic bonding, thermal fusion bonding or the like.

For example, as shown in Fig. 4(c), the absorbent sheet 10c is configured so that, when the superabsorbent polymer resin in the absorbent layers 21 to 23 and 31 to 33 swells, the base sheet 12 is compressed in the thickness direction and deformed, so that crushing o the channels 26 and 27, and 36 and 37 can be prevented.

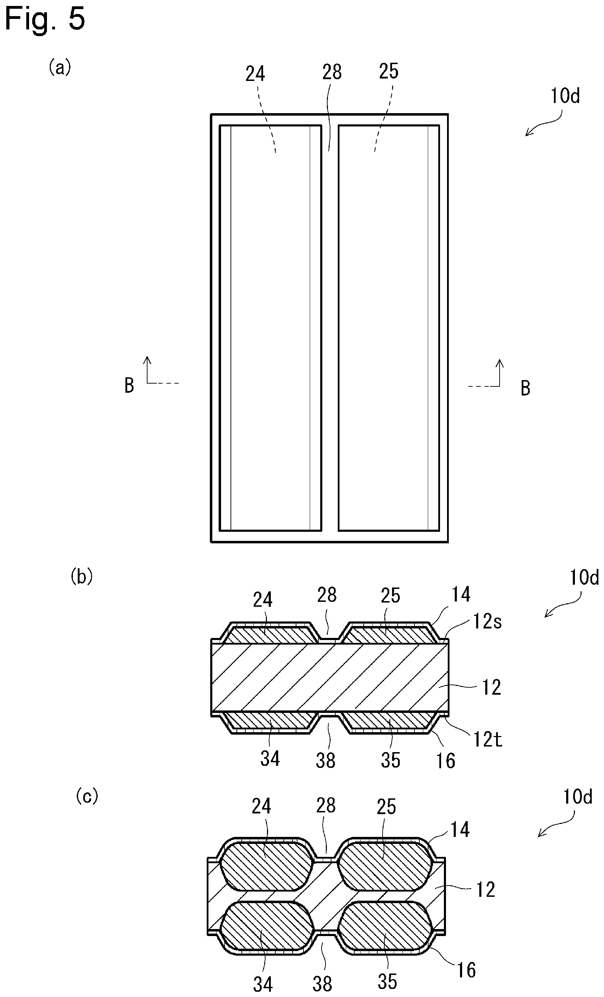

[Fourth modification] Like an absorbent sheet 10d of a fourth modification shown in Fig. 5, one channel 28 may be provided on the one main surface 12s side of the base sheet 12, and one channel 38 may be provided on the other main surface 12t side of the base sheet 12.

On the absorbent sheet 10d, two absorbent layers 24 and 25 are disposed on the one main surface 12s of the base sheet 12 and the channel 28 is formed between the absorbent layers 24 and 25. In addition, two absorbent layers 34 and 35 are disposed on the other main surface 12t of the base sheet 12, and the channel 38 is formed between the absorbent layers 34 and 35.

For example, as shown in Fig. 5(c), the absorbent sheet 10d is configured so that, when the superabsorbent polymer resin in the absorbent layers 24 and 25, and 34 and 35 swells, the base sheet 12 is compressed in the thickness direction and deformed, so that crushing of the channels 28 and 38 can be prevented.

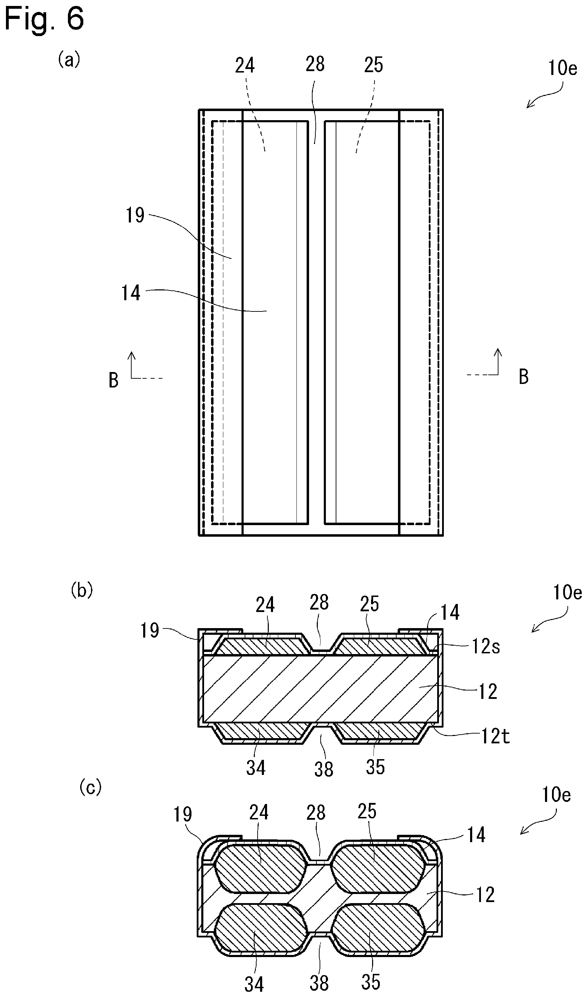

[Fifth modification] Like an absorbent sheet 10e of a fifth modification shown in Fig. 6, the covering sheet 16 of the fourth modification may be changed to the core wrap sheet 19 as in the third modification.

For example, as shown in Fig. 6(c), the absorbent sheet 10e is configured so that, when the superabsorbent polymer resin in the absorbent layers 24 and 25, and 34 and 35 swells, the base sheet 12 is compressed in the thickness direction and deformed, so that crushing of the channels 28 and 38 can be prevented.

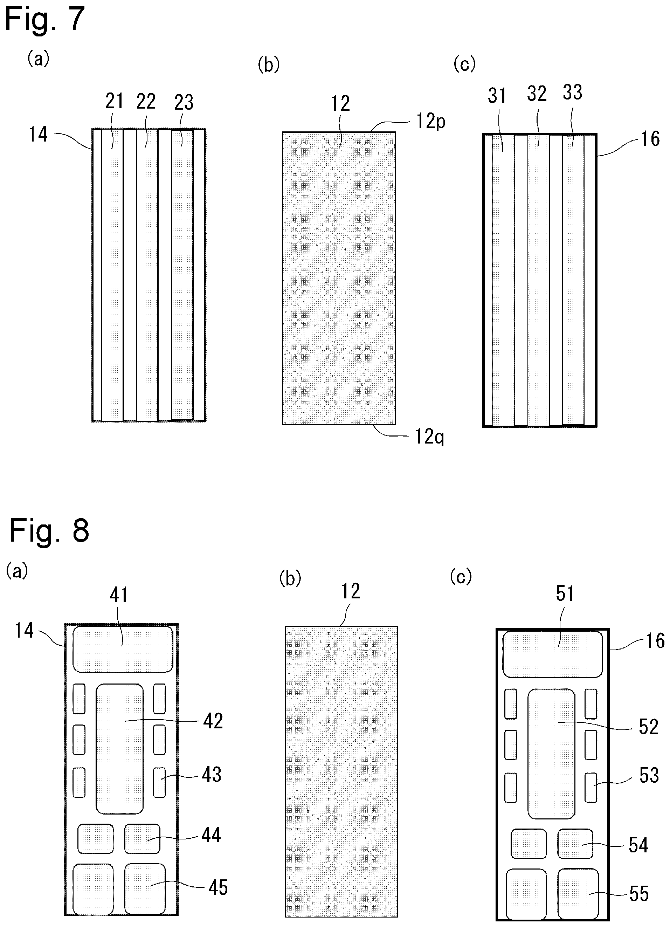

[Sixth modification] Fig. 7(a) is a perspective view where the covering sheet 14 and the absorbent layers 21 to 23 disposed on the one main surface side of the base sheet 12 of the absorbent sheet of a sixth modification are viewed through, Fig. 7(b) is a plan view of the base sheet 12, and Fig. 7(c) is a perspective view where the covering sheet 16 and the absorbent layers 31 to 33 disposed on the other main surface side of the base sheet 12 are viewed through.

As shown in Fig. 7, the absorbent layers 21 to 23 and 31 to 33 may be disposed without any gap between them and the edges 12p and 12q of the base sheet 12.

[Seventh modification] Fig. 8(a) is a perspective view where the covering sheet 14 and absorbent layers 41 to 45 disposed on the one main surface side of the base sheet 12 of the absorbent sheet of a seventh modification are viewed through, Fig. 8(b) is a plan view of the base sheet 12, and Fig. 8(c) is a perspective view where the covering sheet 16 and absorbent layers 51 to 55 disposed on the other main surface side of the base sheet 12 are viewed through.

As shown in Fig. 8, a plurality of absorbent layers 41 to 45 and 51 to 55 having different sizes and configurations may be disposed on the main surfaces of the base sheet 12. Moreover, a plurality of absorbent layers 41 to 45 and 51 to 55 may be disposed with spacing in between in the length direction of the base sheet 12 to form channels extending in the width direction intersecting with the length direction.

The absorbent sheet may be configured by appropriately selecting and combining the configurations disclosed in the first embodiment and the first to seventh modifications. For example, a configuration may be adopted such that only the absorbent layer disposed on one of the one main surface 12s and the other main surface 12t of the base sheet 12 of the absorbent sheets 10b to 10e of the second to fifth modifications is changed as in the seventh modification.

[Summary] The absorbent sheet described above is capable of preventing channel crushing.

The present invention is not limited to the above-described embodiment but may be carried out with various modifications being added.

For example, the shapes of the base sheet and the absorbent layers are not limited to the rectangular form.

### DESCRIPTION OF REFERENCE NUMERALS

10, 10a to 10e Absorbent sheet
12 Base sheet
12s, 12t Main surface
14, 16 Covering sheet
21 to 25 Absorbent layer
31 to 35 Absorbent layer
41 to 45 Absorbent layer
51 to 55 Absorbent layer

## Claims

1. An absorbent sheet comprising:
a base sheet;
a plurality of absorbent layers disposed with spacing in between on a main surface of the base sheet and containing a superabsorbent polymer resin; and
a covering sheet covering the main surface of the base sheet and the absorbent layers,
wherein when the superabsorbent polymer resin in the absorbent layers swells, the base sheet is more easily deformed than the covering sheet.

2. The absorbent sheet according to claim 1, wherein when the superabsorbent polymer resin in the absorbent layers swells, the base sheet is compressed in a thickness direction and deformed.

3. The absorbent sheet according to claim 1 or 2, wherein the base sheet has a lower rebound than the covering sheet.

4. The absorbent sheet according to any one of claims 1 to 3, wherein the covering sheet is bonded to an area of the main surface of the base sheet where the superabsorbent polymer resin is substantially absent.

5. The absorbent sheet according to any one of claims 1 to 4, wherein the lateral stress on the base sheet at 5% swelling is lower than the lateral stress on the covering sheet at 5% swelling.

6. The absorbent sheet according to any one of claims 1 to 5, wherein the base sheet has a bulky structure where pores are formed between fibers.

7. The absorbent sheet according to claim 6, wherein the bulk density of the bulky structure of the base sheet is lower than the bulk density of the covering sheet.

8. The absorbent sheet according to any one of claims 1 to 7, wherein the thickness of the covering sheet is equal to the thickness of the base sheet or is smaller than the thickness of the base sheet.
